# EUROPEAN PATENT APPLICATION

(11) **EP 2 886 645 A1**
(43) Date of publication of application: **24.06.2015**
(21) Application number: 13198798.4
(22) Date of filing: 20.12.2013
(51) Int. Cl.: C12N 5/0783, C07K 16/28

(54) **Expansion of human T cells in vitro by bead-bound conventional anti-CD28 monoclonal antibodies**

(71) Applicant: Julius-Maximilians-Universität Würzburg, 97070 Würzburg (DE)
(72) Inventor: Beyersdorf, Niklas, 97076 Würzburg (DE)
(74) Representative: Isarpatent

(57) **Abstract**

The present invention is directed to an *in vitro* method for the expansion of T cells, a solid bead having a conventional anti-CD28 monoclonal antibody immobilized thereto, and to a kit containing the same. Furthermore, the present invention is directed to the use of a conventional anti-CD28 monoclonal antibody in the *in vitro* expansion of T cells. The *in vitro* method involves reacting T cells from a donor with immobilized conventional anti-CD28 monoclonal antibodies under suitable conditions thereby expanding the T cells.

## Description

### TECHNICAL FIELD OF THE INVENTION

The present invention is directed to an *in vitro* method for the expansion of T cells, a solid bead having a conventional anti-CD28 monoclonal antibody immobilized thereto, and to a kit containing the same. Furthermore, the present invention is directed to the use of a conventional anti-CD28 monoclonal antibody in the *in vitro* expansion of T cells.

### BACKGROUND OF THE INVENTION

### T lymphopenia or functional deficiency of T cells is a frequent medical problem

In several clinical conditions patients may profit from an increase in the numbers of T cells in their body and/or a functional modulation of their T cell compartment. Physical absence of T cells (T lymphopenia) or functional impairment of the T cells can both be a direct consequence of an illness or may be secondary to treatment e.g. in the form of irradiation or chemotherapy.

Lacking T cells means that these patients may experience difficulties containing latent viral infections with viruses like the Epstein Barr Virus, that immune responses to newly encountered pathogens might be severely hampered and that anti-cancer immunity is insufficient. With regard to functional deficits, patients may harbor a numerically normal T cell compartment yet lack T cells of a certain quality like e.g. anti-viral T cells or so-called regulatory T cells which are potent suppressors of autoimmunity.

In the case of gross T cell deficiency, it is a prime therapeutic goal to overcome this lack of T cells as quickly as possible. However, for many forms of T lymphopenia there are no routine protocols for directly enhancing T cell numbers. An exception to this rule are patients undergoing hematopoetic stem cell transplantation followed by so-called donor leukocyte infusions (DLI) during which the patients receive mature T cells. While these T cells may help to quickly reconstitute anti-infective immunity and may elicit the desired graft versus tumor effect in the recipient they may also cause a potentially life-threatening condition called Graft versus Host Disease (GvHD). As the currently available therapeutic options for GvHD are not always sufficient to stop disease progression, studies in humans evaluating adoptive transfers of regulatory T cells to prevent GvHD are ongoing (1, 2).

The infusion of T cells into patients with normal T cell numbers to substitute for a functional deficit within the T cell compartment is today still in development and not part of routine patient care. The most advanced approaches comprise infusion of cancer-specific T cells (3).

### Established protocols for the expansion of human T cells in vitro

With peripheral blood as the main source, access to human T cells is limited and, therefore, expansion of the T cells *in vitro* prior to re-infusion into the patient greatly facilitates their use. This is even more so when not bulk T cells for therapy but rare subpopulations like T cells with specificity for certain viral or cancer antigens or regulatory T cells are needed.

All protocols currently used for antigen-independent expansion of T cells *in vitro* for further clinical use *in vivo* include stimulatory monoclonal antibodies (mAb) against CD3 mimicking what is called 'signal 1' in T cell biology (4, 5). As signal 1 is not sufficient to achieve full T cell activation mAbs to CD28 induce 'signal 2' in the T cells (4, 5). To further enhance T cell proliferation, recombinant interleukin-2 (IL-2) is added to these protocols generating 'signal 3' (6).

The most widely used protocols for *in vitro* expansion of human T cells add monoclonal anti-CD3 antibodies like Okt-3 either alone or together with anti-CD28 monoclonal antibodies (mAb) to peripheral blood mononuclear cells (PBMC) or to purified T cells in the presence or absence of antigen-presenting cells (7-9) or use magnetic beads coated with mAbs with specificity for CD3 and CD28 like the Dynabeads^{®} CD3/CD28 CTS™ from Life Technologies (7, 9, 10). As already mentioned, recombinant human IL-2 is also routinely included in these stimulations.

For some applications, the functionality of the T cells obtained under these conditions may, however, be impaired due to the preferential expansion of regulatory T cells over conventional T cells (8).

The protocols in use for expansion of human T cells *in vitro* suffer from the drawback that stimulation of the T cell receptor complex (TCR) with anti-CD3 mAb increases the susceptibility of the T cells towards apoptosis induction (11). Ideally, protocols for the expansion of T cells *in vitro* would, thus, spare the TCR to avoid this unwanted effect.

So-called superagonistic anti-CD28 mAb (CD28-SA) offer a solution to this problem in that they are capable of fully activating T cells, including regulatory T cells, without the need for anti-CD3 mAb-mediated stimulation of the TCR (12, 13). The disadvantage of CD28-SA like clones TGN1412, ANC28 or BW828, however, is that they show a strong preference towards stimulating memory CD4⁺ T cells (14-16), which led to the unwanted release of pro-inflammatory cytokines in a phase I study *in vivo* (17). Apart from memory CD4⁺ T cells, regulatory T cells are also activated by CD28-SA and this has been shown to translate into protection from autoimmunity in preclinical animal models (18).

The stimulatory potential of non-superagonistic, i.e. conventional, anti-CD28 mAb is by definition only revealed in conjunction with TCR stimulation. Attempts to use these antibodies in the absence of TCR stimulation for *in vitro* expansion of T cells have not been very successful so far with reports of a conventional anti-CD28 mAb bound to plastic inducing proliferation in 5% of naïve human T cells (19) and another conventional anti-CD28 mAb, clone 9.3, selectively inducing proliferation of human memory T cells and this only when recombinant human IL-2 was added (14).

### SUMMARY OF THE INVENTION

In the prior art, expansion of human T cells *in vitro* is either already in use or has the potential to become an essential component of many immunotherapeutic approaches. The most widely used protocols for *in vitro* expansion of human T cells rely on monoclonal antibodies (mAb) with specificity for CD3 and the co-stimulatory protein CD28 as well as the cytokine interleukin-2. Drawbacks of these protocols either are that stimulation of CD3, i.e. the T cell receptor complex, is associated with induction of apoptosis in T cells or that only subpopulations of CD4⁺ T cells are expanded. The disadvantage of CD28-SA is that they show a strong preference towards stimulating memory CD4⁺ T cells leading to the unwanted release of pro-inflammatory cytokines.

Therefore, it is a problem underlying the present invention to provide an *in vitro* method for T cell expansion which does not require TCR engagement. It is a further problem underlying the invention to provide an *in vitro* method for T cell expansion which avoids the use of superagonistic anti-CD28 mAbs and/or of monoclonal antibodies with specificity for CD3. It is a still further problem to provide an *in vitro* method for T cell expansion which induces unbiased expansion of CD4⁺ T cell subsets.

The present invention solves the above problems by using a novel protocol for polyclonal expansion of total human T cells *in vitro* using conventional, i.e. non-superagonistic anti-CD28 mAb, that, when immobilized on beads, induce T cell proliferation.

The invention described herein constitutes a novel form of polyclonal expansion of human T cells *in vitro* using conventional, i.e. non-superagonistic, anti-CD28 mAb immobilized on beads, for example, magnetic beads. This method allows for T cell expansion *in vitro* without TCR engagement, avoids the use of superagonistic anti-CD28 mAb's and of monoclonal antibodies with specificity for CD3, and induces unbiased expansion of CD4⁺ T cell subsets.

### BRIEF DESCRIPTION OF THE FIGURES

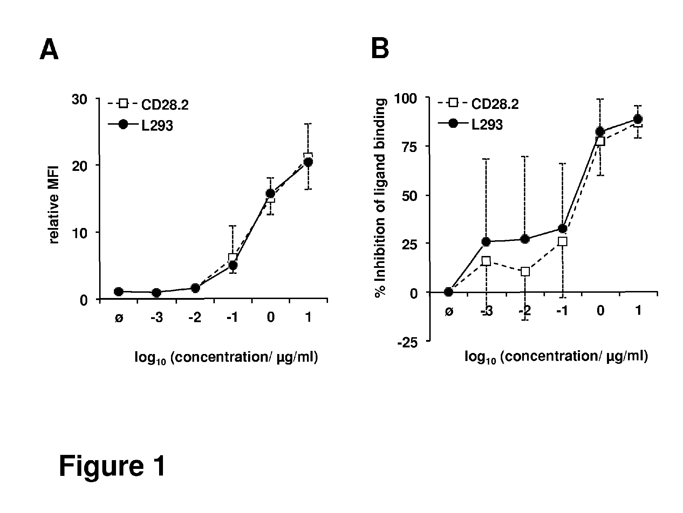

Figure 1 shows that mAb CD28.2 and L293 (both Becton Dickinson) themselves bind to the CD28 molecule on gated human CD4⁺ T cells and that saturating amounts of these mAb (≥ 1 µg/ ml) efficiently block binding of CD80-Ig to CD28.

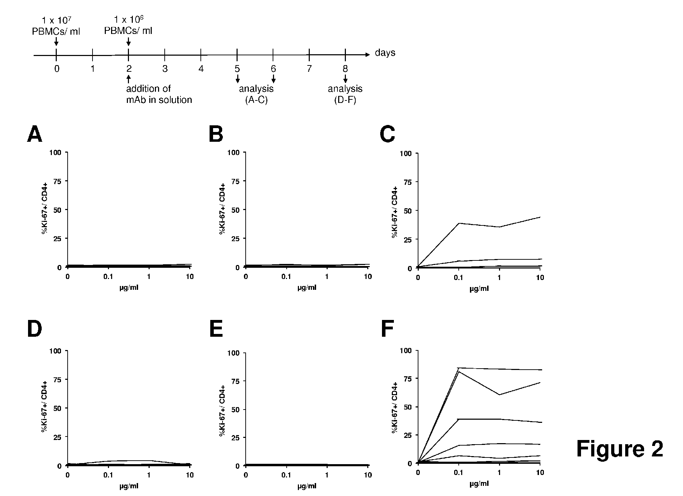

Figure 2: PBMC were cultured at 1 x 10⁷ PBMC/ ml (24-well plate; total volume: 1.5 ml) for two days before diluting them to 1 x 10⁶ PBMC/ ml (96-well round bottom plate) and addition of the stimulating reagents (scheme). As a read-out for proliferation expression of Ki-67 in CD4⁺ T cells on days three/four (Fig. 2A-C) and six after initiation of the cultures (Fig. 2D-F) was measured.

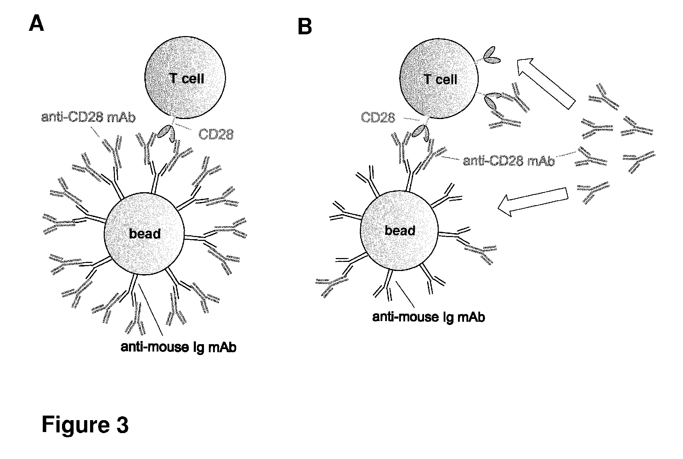

Figure 3: As depicted in Fig. 3A the beads were either loaded with anti-CD28 mAb followed by removal of unbound mAb and incubation of the beads with PBMC containing the targeted T cells or the beads were directly mixed with PBMC and then the anti-CD28 mAb was added in solution (Fig. 3B).

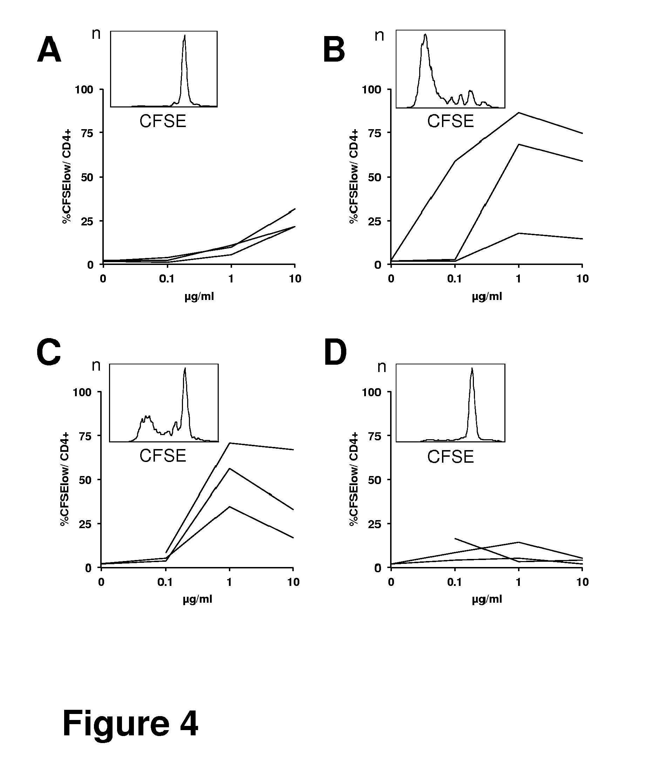

Figure 4: The beads were loaded with CD28.2 (Fig. 4A) or L293 (Fig. 4B) or the mAb's were added in solution to the suspension of PBMC and beads (Fig. 4C: CD28.2, Fig. 4D: L293). The antibody amounts used during the coating step or in solution are indicated. After six days the degree of CFSE dilution among CD4⁺ T cells reflecting cell proliferation was analyzed.

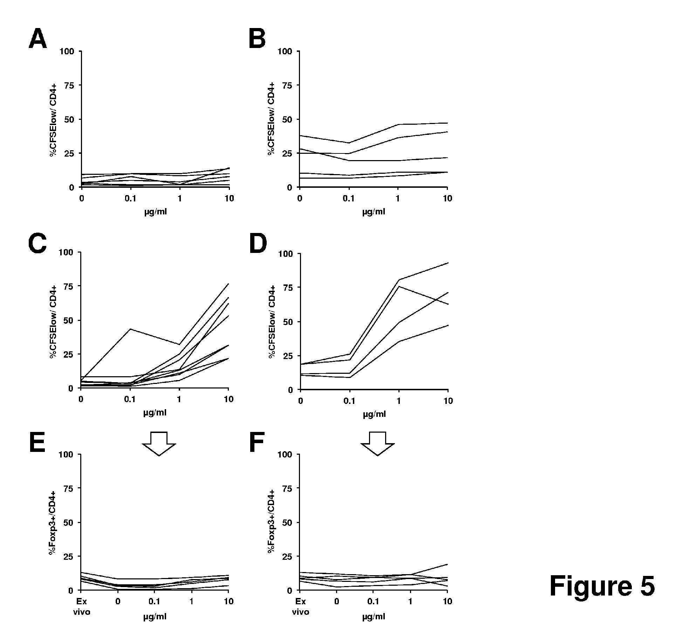

Figure 5: In the experiments summarized in Fig. 5A mAb CD28.2 was added without IL-2 to CFSE-labeled PBMC which were not pre-cultured at high density. Addition of 10⁻⁶ M IL-2 (Proleukin^{®}, Novartis) by itself, i.e. without mAb CD28.2, induced some proliferation in CD4⁺ T cells independent of the amount of mAb CD28.2 added (Fig. 5B). Soluble CD28.2 and Dynabeads^{®} Pan Mouse IgG induced proliferation of CD4⁺ T cells (Fig. 5C) which was increased upon addition of IL-2 (Fig. 5D). Analyzing the frequencies of Foxp3⁺ cells among CD4⁺ T cells in the experiments summarized in Fig. 5C and Fig. 5D revealed that stimulation with mAb CD28.2 at concentrations of 10 µg/ ml maintained *ex vivo* frequencies of Foxp3⁺ cells among CD4⁺ T cells (Fig. 5E), as did 10⁻⁶ M IL-2 (Fig. 5F). MAb CD28.2 and IL-2 did, however, not synergize in increasing the frequencies of Foxp3⁺ cells among CD4⁺ T cells (Fig. 5F).

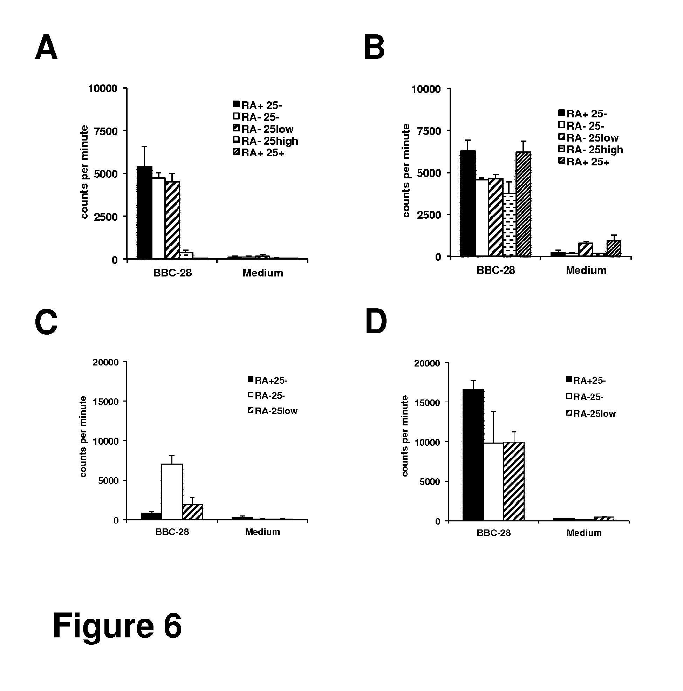

Figure 6: In experiment I (Fig. 6A, B) BBC-28 induced proliferation of naïve CD4⁺ T cells (CD45RA⁺ CD25⁻), memory CD4⁺ T cells (CD45RA⁻ CD25⁻) and CD25^{low} memory CD4⁺ T cells (CD45RA⁻ CD25^{low}) in the absence of exogenously added IL-2 (Fig. 6A). In the presence of IL-2 also resting T_{reg} cells (CD45RA⁺ CD25⁺) and activated T_{reg} cells (CD45RA⁻ CD25^{high}) proliferated (Fig. 6B). The PBMC used in experiment II (Fig. 6C, D) had very low frequencies of resting and activated T_{reg} cells so that we could only analyze the three remaining subsets. In the absence of exogenously added IL-2, primarily CD4⁺ memory T cells (CD45RA⁻ CD25⁻ and CD45RA⁻ CD25^{low}) proliferated (Fig. 6C). Addition of IL-2 preferentially enhanced the BBC-28-induced proliferation of naïve T cells (CD45RA⁺ CD25⁻) which in the presence of exogenously added IL-2 proliferated more strongly than the memory T cells (Fig. 6D).

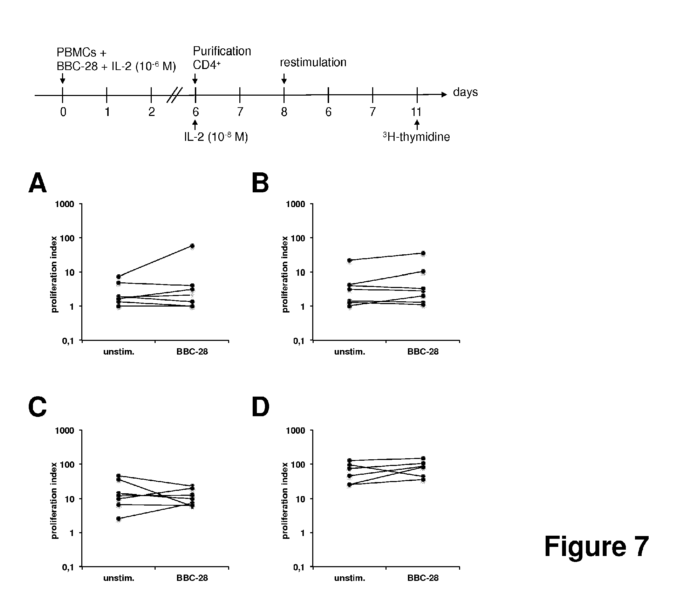

Figure 7: The response of BBC-28 plus IL-2 (10⁻⁶ M Proleukin^{®})-expanded human CD4⁺ T cells towards stimulation with antigenic recall antigens such as Tetanus and Diphtheria toxoid (Chiron Behring, Fig. 7A) and purified protein derivate of *Mycobacterium tuberculosis* (PHARMORE, Fig. 7B) was evaluated. In addition, also proliferative responses after anti-CD3 mAb HIT3a (Becton Dickinson, Fig. 7C) and after anti-CD3 mAb HIT3a and anti-CD28 mAb CD28.2 stimulation (Fig. 7D) were tested and compared to those of thawed but otherwise un-manipulated CD4⁺ T cells of the same donor.

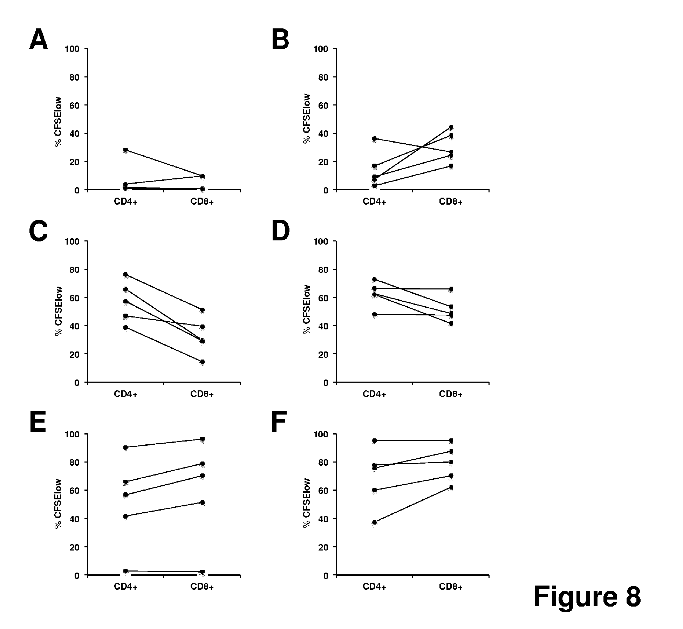

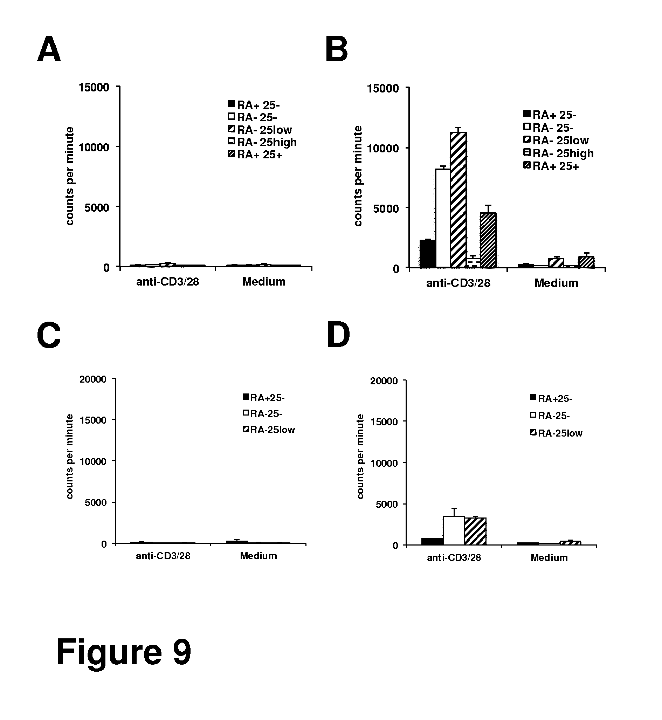

Figure 8: CD4⁺ T cells show a better response towards BBC-28 stimulation than CD8⁺ T cells. Human PBMC were isolated and labeled with CFSE as described in section 3. The cells were then stimulated with BBC-28 without (C) or with (D) 10⁻⁶ M IL-2 (Proleukin^{®}, Novartis). Alternatively, Dynabeads^{®} M-270 Epoxy coated with 20 µg/ ml anti-CD3 mAb (clone UCHT1, Becton Dickinson) and 10 µg/ ml CD28.2 according to the manufacturer's instructions were used (E without and F with IL-2). To control for mAb-induced proliferation PBMC were incubated with uncoated Dynabeads^{®} Pan Mouse IgG (A: without and B with IL-2). After six days in culture the degree of CFSE dilution among CD4⁺ and CD8⁺ T cells was determined. Figure 9: BBC-28 elicits a more rigorous and less biased proliferative response in purified CD4⁺ T cells than anti-CD3/CD28-coated beads. The data depicted in this supplement were obtained by analyzing parallel cultures of those shown in Figure 6. Instead of BBC-28 the purified CD4⁺ T cell subpopulations were stimulated with anti-CD3/CD28-coated beads either in the absence (A and C) or presence (B and D) of 10⁻⁶ M IL-2. The data show that the overall proliferative response of purified CD4⁺ T cells was weaker upon anti-CD3/CD28-coated bead stimulation than upon BBC-28 stimulation. Moreover, the anti-CD3/CD28-coated beads, but not BBC-28, preferentially induced proliferation of memory T cells (CD45RA⁻ CD25⁻ and CD45RA⁻ CD25^{low}) as well as resting T_{reg} cells (CD45RA⁺ CD25⁺) over naïve T cells (CD45RA⁺ CD25⁻).

### DETAILED DESCRIPTION OF THE INVENTION

In a first aspect, the present invention is directed to an *in vitro* method for the expansion of T cells comprising the steps of
a) providing conventional anti-CD28 monoclonal antibodies immobilized on solid beads;
b) providing T cells from a donor;
c) reacting the T cells with the immobilized conventional anti-CD28 monoclonal antibodies under suitable conditions thereby expanding the T cells.

Surprisingly, the use of conventional anti-CD28 monoclonal antibodies immobilized to solid beads as defined below in an *in vitro* method for the expansion of T cells led to a remarkable expansion of those T cells in complete absence of anti-CD3 antibodies and superagonistic anti-CD28 monoclonal antibodies. Further, the engagement of TCR's could be avoided and unbiased expansion of T cell, in particular CD 4⁺ T cell, subsets could be achieved.

One of the key requirements of the present invention is the use of so-called "conventional" anti-CD28 monoclonal antibodies. These antibodies can be defined as follows:
"Conventional" anti-CD28 mAb's can be identified by analyzing their capacity to block binding of a soluble ligand for CD28, CD80-lg, to CD28 expressed by CD4⁺ T cells. Thus, a conventional anti-CD28 mAb is characterized by binding an epitope on the CD28 molecule close to the binding site of the ligands of CD28, CD80 and CD86, and therefore outside the so-called C"D loop, which has been identified as a key feature of conventional anti-CD28 mAb (20).

In order to clearly distinguish conventional from so-called superagonistic anti-CD28 antibodies, the method recently published by Römer et al. (16) which increases the sensitivity of human T cells isolated from peripheral blood towards activating reagents and, thus, has the potential to reveal superagonistic activity of anti-CD28 mAb, may be used. This is also called the RESTORE protocol.

Basically, conventional anti-CD28 mAb can be distinguished from those having superagonistic activity in that binding of the (conventional) anti-CD28 mAb to an epitope on the CD28 molecule close to the ligand binding site precludes superagonistic activity of an anti-CD28 mAb.

Further information on how to distinguish conventional from superagonistic anti-CD28 mAb can be found in (22) which is incorporated herein by reference. As noted therein, superagonistic anti-CD28 antibodies bind to a lateral, membrane-proximal loop of the molecule (C"D loop).

The amino acid sequence of human CD28 is known under the accession no. NM-006139. The C"D loop of CD28 comprises amino acids 52 to 66 of the above CD28 sequence (see also Ostrov, D. A. et al.; Science (2000), 290:816-819). The term C"D loop will in the following also comprise any partial sequences thereof.

The term "antibody" is used herein for intact antibodies as well as antibody fragments, which have a certain ability to selectively bind to an epitope. Such fragments include, without limitations, Fab, F(ab')2 and Fv antibody fragments. The term "epitope" means any antigen determinant of an antigen, to which the paratope of an antibody can bind. Epitope determinants usually consist of chemically active surface groups of molecules (e.g. amino acid or sugar residues) and usually display a three-dimensional structure as well as specific physical properties. The term antibody also comprises (complete) chimeric and humanized antibodies.

The production of polyclonal antibodies is commonly known. Detailed protocols can be found for example in Green et al, Production of Polyclonal Antisera, in Immunochemical Protocols (Manson, editor), pages 1-5 (Humana Press 1992) and Coligan et al, Production of Polyclonal Antisera in Rabbits, Rats, Mice and Hamsters, in Current Protocols In Immunology, section 2.4.1 (1992). In addition, the expert is familiar with several techniques regarding the purification and concentration of polyclonal antibodies (Coligan et al, Unit 9, Current Protocols in Immunology, Wiley Interscience, 1994).

The production of monoclonal antibodies such as the above anti-CD28 monoclonal antibody, is as well commonly known. Examples include the hybridoma method (Kohler and Milstein, 1975, Nature, 256:495-497, Coligan et al., section 2.5.1-2.6.7; and Harlow et al., Antibodies: A Laboratory Manual, page 726 (Cold Spring Harbor Pub. 1988).), the trioma technique, the human B-cell hybridoma technique (Kozbor et al., 1983, Immunology Today 4:72), and the EBV-hybridoma technique to produce monoclonal antibodies (Cole, et al., 1985, in Monoclonal Antibodies and Cancer Therapy, Alan R. Liss, Inc., pp. 77-96).

A further key requirement of the method according to the present invention is that the anti-CD28 monoclonal antibody is immobilized on solid beads. Antibody immobilization onto solid surfaces has been studied extensively for a number of applications including immunoassays, biosensors, and affinity chromatography. Solid beads such as colloidal beads of polyacrolein, polyaldehyde, polystyrene or latex beads are well known in the prior art. Antibodies may be immobilized via covalent binding for example of primary amine groups to N-hydroxysuccinimide (NHS)-activated sepharose beads or by the covalent reaction of hydrazinonicotinamide-modifed antibody with formylbenzamide-modified magnetic beads.

Nonmagnetic beads suitable for use in the provided methods, such as polymer beads, are commercially available and techniques for attaching an antibody to such beads are well known in the art. Beads generally are essentially spherical particles of a size between about 0.5 and 10 µm. In a preferred embodiment, the beads used in the method according to the present invention have a diameter of about preferably 4-5 µm.

The use of magnetism in cell isolation techniques is well known and magnetic surfaces for use in such techniques are commercially available. Magnetic or magnetizable surfaces, such as beads, for use in the present method can be without limitation, magnetic, paramagnetic, or superparamagnetic. The term "magnetic" as used herein means that the bead is capable of having a magnetic moment imparted to it when placed in a magnetic field, and thus is displaceable under the action of that field. Accordingly, magnetic particles may readily be removed from other components of a sample by magnetic aggregation.

Magnetic beads suitable for use in the methods provided are of a variety of compositions including, but not limited to, fine grains of iron oxides dispersed throughout the interior of a polymer particle, silanized beads of magnetic iron oxides, and silanized beads of magnetic porous glass. Polymer compositions of magnetic polymer particles include, for example, polystyrene, cellulose, and latex. Commercially available magnetic beads are available with surfaces modified so as to allow for direct or indirect attachment of molecules, such as an antibody, to the bead surface. Surface modifications of such beads include, but are not limited to, immobilized antibodies such as, without limitation, secondary antibodies; immobilized protein A, immobilized protein G, immobilized streptavidin, immobilized avidin, immobilized biotin, immobilized oligo (dT), and having end groups such as, without limitation, -COOH, -NH₂, -OH, epoxy-, tosyl- activated, hydrazide, and glyceryl.

A preferred group of beads for use in the present invention are magnetic beads, preferably superparamagnetic polystyrene beads.

In a preferred embodiment of the invention, the conventional anti-CD28 monoclonal antibody is indirectly immobilized to the beads, preferably via another antibody. For example, the beads are coated with a monoclonal anti-mouse IgG antibody (such as those commercially available under the tradename Dynabeads^{®} Pan Mouse IgG manufactured by Dynal^{®} Biotech). Dynabeads^{®} Pan Mouse IgG are uniform, superparamagnetic polystyrene beads (4.5 µm diameter) coated with a monoclonal human anti-mouse IgG antibody. The antibody coated onto Dynabeads^{®} recognizes all mouse IgG subclasses and is Fc specific.

The use of an indirect immobilization has the following advantages: a uniform binding of the anti-CD28 monoclonal mAb's is guaranteed; further, indirect binding of the anti-CD28 mAb to the bead via a linker such as the human anti-mouse IgG-Fc mAb increases the steric flexibility of the immobilized anti-CD28 mAb, which should in turn facilitate interaction of the mAb with the CD28 molecule. However, the present invention is not restricted to an antibody as a linker.

The method according to the present invention can be used to expand all types of T cells, for example CD8⁺ T cells and/or CD4⁺ T cells. In a preferred embodiment of the invention, the T cells expanded by the method of the invention are CD4⁺ T cells.

According to the present invention, the T cells provided in step b) are derived from peripheral blood of a human donor. That is to say, a body liquid is taken from a patient, comprising blood comprising T lymphocytes or precursor cells thereof such as PBMC's (Peripheral Blood Mononuclear Cells). Subsequently, the body liquid, possibly after a processing step, is reacted with an immobilized anti-CD28 monoclonal antibody, and the thus treated body liquid is again administered to the patient.

The galenic production of the various forms of administration is well known to experts and need not be explained in more detail here. The only requirement is that the T cells expanded in step c) are further processed to a form suitable for parenteral, preferably intravenous, administration to said donor.

The expanded CD4⁺ T cells may be used in the treatment of diseases with pathologically reduced CD4 T cell figures, such as AIDS or in stem cell transplantations following chemotherapy of leukemic diseases, for potentiating and/or qualitative influence of immune reactions in protective inoculations and/or in order to influence the quality of T cell reactions, in particular to influence the production of various effector molecules, for example cytokines and chemokines and their receptors, e.g. in auto-immune diseases. Similar to the *in vivo* application of Dynabeads^{®} coated with reagents stimulating the T cell receptor and CD28 in mice (Ugel et al., 2009, Cancer Research 69:9376), application of a drug containing conventional anti-CD28-mAb coated Dynabeads^{®} Pan Mouse IgG (BBC-28) can also activate and expand T cells in humans *in vivo.* Recognition of the CD28 molecule by BBC-28 and efficient stimulation of CD28 due to the immobilization of the anti-CD28 mAb on beads induces the expansion of T cells *in vivo.*

A preferred example of an anti-CD28 monoclonal antibody of the present invention is CD28.2 or L293. CD28.2 is commercially available from BD Biosciences, Santa Cruz Biotechnology, Affymetrix or Abcam, L293 from Fisher Scientific or BD Biosciences.

CD28.2 has been first described in Nunès et al., 1993, International Immunology 5:11 and L293 in Azuma et al., 1992, The Journal of Experimental Medicine 175:353.

In a further aspect, the present invention is directed to a solid bead having a conventional anti-CD28 monoclonal antibody immobilized thereto, wherein the bead is free of anti-CD3 antibodies. That is to say, the solid beads incorporate anti-CD28 antibodies as the only active principle whereas is not excluded that antibodies used for indirect immobilization are bound to the bead as well. Thus, the present invention provides for the use of a conventional anti-CD28 monoclonal antibody in the *in vitro* expansion of T cells, where the conventional anti-CD28 monoclonal antibody is the only anti-CD antibody used.

For example, the bead may be coated with an anti-mouse Ig monoclonal antibody to which the conventional anti-CD28 monoclonal antibody is bound (see the illustration in Figure 3). Thus, a coated bead according to the invention may consist of (or consist essentially of) a solid bead, an anti-mouse Ig monoclonal antibody and a conventional anti-CD28 monoclonal antibody.

In a further aspect, the present invention is directed to a kit consisting essentially of a bead as defined above.

The following Examples are offered for the sake of illustrating the invention and should not be construed as limiting the invention.

### EXAMPLES

### 1 Characterization of monoclonal anti-CD28 antibodies used for T cell stimulation

### 1.1 MAbs CD28.2 and L293 block binding of CD80-Ig to CD28

We first evaluated whether the two monoclonal antibodies used for this study fulfill the criteria for being classified as 'conventional' anti-CD28 mAb by analyzing their capacity to block binding of a soluble ligand for CD28, CD80-Ig (R&D Systems), to CD28 expressed by CD4⁺ T cells contained in peripheral blood mononuclear cells (PBMC) isolated from healthy blood donors (material obtained anonymously from the Department of Transfusion Medicine, University Hospital Würzburg). Figure 1A shows that mAb CD28.2 and L293 (both Becton Dickinson) themselves bind to the CD28 molecule on gated human CD4⁺ T cells and that saturating amounts of these mAb (≥ 1 µg/ ml) efficiently block binding of CD80-Ig to CD28. Therefore, both mAb bind an epitope on the CD28 molecule close to the binding site of the ligands of CD28, CD80 and CD86, and therefore outside the so-called C"D loop, which has been identified as a key feature of conventional anti-CD28 mAb, including the antihuman CD28 mAb 7.3B6 (20).

### 1.2 No mitogenic signal induced by soluble mAb CD28.2 and L293 in CD4⁺ T cells pre-cultured at high density

To further corroborate that mAb CD28.2 and L293 are bona fide conventional anti-CD28 mAb we tested both mAb with the method recently published by Römer et al. (16) which increases the sensitivity of human T cells isolated from peripheral blood towards activating reagents and has, thus, the potential to reveal superagonistic activity of anti-CD28 mAb. In accordance with Römer et al. we first cultured PBMC at 1 x 10⁷ PBMC/ ml (24-well plate; total volume: 1.5 ml) for two days before diluting them to 1 x 10⁶ PBMC/ ml (96-well round bottom plate) and addition of the stimulating reagents (scheme Fig. 2). As a read-out for proliferation we measured expression of Ki-67 in CD4⁺ T cells on days three/four (Fig. 2A-C) and six after initiation of the cultures (Fig. 2D-F). While addition of the anti-CD3 mAb UCHT1 (Becton Dickinson) in solution induced proliferation indicated by an increase in the frequency of Ki-67⁺ cells among CD4⁺ T cells (Fig. 2C and F) neither mAb CD28.2 (Fig. 2A and D) nor mAb L293 (Fig. 2B and E) were mitogenic. These functional data, thus, confirmed that mAb CD28.2 and L293 adhere to the rule that binding of an epitope on the CD28 molecule close to the ligand binding site precludes superagonistic activity of an anti-CD28 mAb.

### 2 Loading of conventional anti-CD28 mAb onto Dynabeads^{®} Pan Mouse IgG

As the binding characteristics of mAb CD28.2 and L293 to the CD28 molecule and the lack of mitogenic activity in solution identified them as conventional anti-CD28 mAb we sought to induce a mitogenic signal in T cells by immobilizing them on superparamagnetic Dynabeads^{®}. We used Dynabeads^{®} Pan Mouse IgG (Life Technologies) to immobilize the anti-CD28 mAb. According to the manufacturer's product description these beads are pre-coated with a monoclonal human antibody recognizing an epitope in the Fc part of mouse IgG.

As depicted in Fig. 3A we either loaded the beads with anti-CD28 mAb followed by removal of unbound mAb and incubation of the beads with PBMC containing the targeted T cells or we directly mixed the beads and PBMC and then added the anti-CD28 mAb in solution (Fig. 3B).

The Dynabeads^{®} Pan Mouse IgG appeared to be particularly promising for our goal as the monoclonal human anti-mouse IgG-Fc mAb would guarantee uniform binding of the anti-CD28 mAb to the beads in a way that would not inhibit interaction of the antigen binding site of the anti-CD28 mAb with the CD28 molecule. Moreover, indirect binding of the anti-CD28 mAb to the bead via the human anti-mouse IgG-Fc mAb increases the steric flexibility of the 'immobilized' anti-CD28 mAb, which should in turn facilitate interaction of the mAb with the CD28 molecule.

### 3 Bead-bound conventional anti-CD28 mAb induce proliferation of human CD4⁺ T cells in vitro

To assess whether immobilization of the conventional anti-CD28 mAb on Dynabeads^{®} Pan Mouse IgG would be sufficient to induce proliferation of primary human T cells *in vitro,* we isolated PBMC from health blood donors, labeled them with the dye carboxyfluorescein succinimidyl ester diacetate (CFSE) and co-incubated them at 1 x 10⁶ cells/ ml (12-well plate; total volume: 4 ml) with conventional anti-CD28-mAb coated Dynabeads^{®} Pan Mouse IgG (BBC-28) at a bead to cell ratio of 4.5:1. The beads were coated according to the manufacturer's instructions and the antibody amounts used during the coating step are indicated in Fig. 4. To ensure equal amounts of protein on the bead surface, mAb coating was followed by incubation of the beads with normal mouse immunoglobulin (20 µg/ ml).

After six days we analyzed the degree of CFSE dilution among CD4⁺ T cells reflecting cell proliferation. As depicted in Fig. 4A mAb CD28.2 was a poor inducer of T cell proliferation when directly coated to the beads (cf. scheme in Fig. 3A), while the opposite was true for mAb L293 (Fig. 4B). In contrast, adding CD28.2 in solution together with Dynabeads^{®} Pan Mouse IgG induced robust proliferation of CD4⁺ T cells (Fig. 4B; scheme Fig. 3B). Under these conditions mAb L293 did not induce proliferation of CD4⁺ T cells (Fig. 4D). The inserts show representative CFSE dilution profiles of CD4⁺ T cells cultured for six days in the presence of beads coated with anti-CD28 mAb at a concentration of 1 µg/ ml.

The present data, thus, show that BBC-28 are capable of inducing proliferation in the majority of human CD4⁺ T cells in vitro. With respect to the degree of immobilization required for a conventional anti-CD28 mAb to induce T cell proliferation we, further, identified two classes of conventional anti-CD28 mAb.

### 4 Addition of recombinant IL-2 supports the proliferation of human CD4⁺ T cells induced by BBC-28 in vitro

For immunotherapeutic approaches large-scale expansion of T cells is often required. Therefore, we analyzed whether addition of IL-2 would improve proliferation of BBC-28-stimulated CD4⁺ T cells and whether including IL-2 would increase the frequencies of Foxp3⁺ regulatory T cells among CD4⁺ T cells.

In the experiments summarized in Fig. 5A we added mAb CD28.2 without IL-2 to CFSE-labeled PBMC which we had not pre-cultured at high density. As expected, mAb CD28.2 induced no proliferation, as read out by CFSE dilution on day six after initiation of the cultures, of CD4⁺ T cells under these conditions. Addition of 10⁻⁶ M IL-2 (Proleukin^{®}, Novartis) by itself, i.e. without mAb CD28.2, induced some proliferation in CD4⁺ T cells independent of the amount of mAb CD28.2 added (Fig. 5B). As already described, soluble CD28.2 and Dynabeads^{®} Pan Mouse IgG (scheme Fig. 3B) induced proliferation of CD4⁺ T cells (Fig. 5C) which was increased upon addition of IL-2 (Fig. 5D). Analyzing the frequencies of Foxp3⁺ cells among CD4⁺ T cells in the experiments summarized in Fig. 5C and Fig. 5D revealed that stimulation with mAb CD28.2 at concentrations of 10 µg/ ml maintained *ex vivo* frequencies of Foxp3⁺ cells among CD4⁺ T cells (Fig. 5E), as did 10⁻⁶ M IL-2 (Fig. 5F). MAb CD28.2 and IL-2 did, however, not synergize in increasing the frequencies of Foxp3⁺ cells among CD4⁺ T cells (Fig. 5F).

These data show that the addition of IL-2 to stimulations of human T cells with BBC-28 quantitatively increases the amount of proliferation induced without increasing the frequencies of Foxp3⁺ cells among CD4⁺ T cells.

### 5 BBC-28 stimulation is capable of activating and expanding all CD4+ T cell subpopulations in vitro

To determine which CD4⁺ T cell subpopulations are expanded by BBC-28, we sorted PBMC of blood donors according to CD4, CD45RA and CD25 expression (21). We then stimulated the subpopulations (5 x 10⁴ cells per well; triplicates) with mAb CD28.2 (10 µg/ ml) in solution plus Dynabeads^{®} Pan Mouse IgG at a bead to cell ratio of 5:1 in the presence or absence of 10⁻⁶ M IL-2. The cultures were pulsed with 3H-thymidine for the last 16 hours of the three-day culturing period. In experiment I (Fig. 6A, B) BBC-28 induced proliferation of naïve CD4⁺ T cells (CD45RA⁺ CD25⁻), memory CD4⁺ T cells (CD45RA⁻ CD25⁻) and CD25^{low} memory CD4⁺ T cells (CD45RA⁻ CD25^{low}) in the absence of exogenously added IL-2 (Fig. 6A). In the presence of IL-2 also resting T_{reg} cells (CD45RA⁺ CD25⁺) and activated T_{reg} cells (CD45RA⁻ CD25^{high}) proliferated (Fig. 6B). The PBMC used in experiment II (Fig. 6C, D) had very low frequencies of resting and activated T_{reg} cells so that we could only analyze the three remaining subsets. In the absence of exogenously added IL-2, primarily CD4⁺ memory T cells (CD45RA⁻ CD25⁻ and CD45RA⁻ CD25^{low}) proliferated (Fig. 6C). Addition of IL-2 preferentially enhanced the BBC-28-induced proliferation of naïve T cells (CD45RA⁺ CD25⁻) which in the presence of exogenously added IL-2 proliferated more strongly than the memory T cells (Fig. 6D).

These data show that BBC-28 stimulation is sufficient to induce proliferation in highly purified CD4⁺ T cells with no overt bias towards a certain subpopulation.

### 6 CD4⁺ T cells retain reactivity towards recall antigens after expansion with BBC-28 and IL-2

In order to be able to assess whether BBC-28 plus IL-2 (10⁻⁶ M Proleukin^{®})-expanded human CD4⁺ T cells would, indeed, be suitable for immunotherapy we evaluated their response towards stimulation with antigenic recall antigens such as Tetanus and Diphtheria toxoid (Chiron Behring, Fig. 7A) and purified protein derivate of Mycobacterium tuberculosis (PHARMORE, Fig. 7B). In addition, we also tested proliferative responses after anti-CD3 mAb HIT3a (Becton Dickinson, Fig. 7C) and after anti-CD3 mAb HIT3a and anti-CD28 mAb CD28.2 stimulation (Fig. 7D) and compared them to those of thawed but otherwise un-manipulated CD4⁺ T cells of the same donor. As depicted in the scheme, we rested the pre-activated CD4⁺ T cells in medium containing 10⁻⁸ M IL-2 for two days before we re-stimulated them in the presence of syngeneic irradiated PBMC as antigen-presenting cells. The proliferation index was calculated by dividing the counts per minute obtained after harvesting the stimulated T cells by the counts per minute detected within the same cell preparation after culture in medium only (3H-thymidine pulsing interval: last 16 h of the 72 h culturing period).

The data show that BBC-28-expanded CD4⁺ T cells mounted equally high responses upon antigenic recall and anti-CD3 ± anti-CD28 mAb stimulation as did un-manipulated CD4⁺ T cells.

### References

1. Edinger, M., and Hoffmann, P. 2011. Regulatory T cells in stem cell transplantation: strategies and first clinical experiences. Curr Opin Immunol 23:679-684.
2. Tang, Q., Bluestone, J.A., and Kang, S.M. 2012. CD4(+)Foxp3(+) regulatory T cell therapy in transplantation. J Mol Cell Biol 4:11-21.
3. June, C.H. 2007. Principles of adoptive T cell cancer therapy. J Clin Invest 117:1204-1212.
4. Bretscher, P., and Cohn, M. 1970. A theory of self-nonself discrimination. Science 169:1042-1049.
5. Bretscher, P.A. 1999. A two-step, two-signal model for the primary activation of precursor helper T cells. Proc Natl Acad Sci U S A 96:185-190.
6. Malek, T.R., and Bayer, A.L. 2004. Tolerance, not immunity, crucially depends on IL-2. Nat Rev Immunol 4:665-674.
7. Hoffmann, P., Eder, R., Kunz-Schughart, L.A., Andreesen, R., and Edinger, M. 2004. Large-scale in vitro expansion of polyclonal human CD4(+)CD25high regulatory T cells. Blood 104:895-903.
8. Mesel-Lemoine, M., Cherai, M., Le Gouvello, S., Guillot, M., Leclercq, V., Klatzmann, D., Thomas-Vaslin, V., and Lemoine, F.M. 2006. Initial depletion of regulatory T cells: the missing solution to preserve the immune functions of T lymphocytes designed for cell therapy. Blood 107:381-388.
9. Hoffmann, P., Eder, R., Boeld, T.J., Doser, K., Piseshka, B., Andreesen, R., and Edinger, M. 2006. Only the CD45RA+ subpopulation of CD4+CD25high T cells gives rise to homogeneous regulatory T-cell lines upon in vitro expansion. Blood 108:4260-4267.
10. Porter, D.L., Levine, B.L., Bunin, N., Stadtmauer, E.A., Luger, S.M., Goldstein, S., Loren, A., Phillips, J., Nasta, S., Perl, A., et al. 2006. A phase 1 trial of donor lymphocyte infusions expanded and activated ex vivo via CD3/CD28 costimulation. Blood 107:1325-1331.
11. Kerstan, A., and Hunig, T. 2004. Cutting edge: distinct TCR- and CD28-derived signals regulate CD95L, Bcl-xL, and the survival of primary T cells. J Immunol 172:1341-1345.
12. Hunig, T., and Dennehy, K. 2005. CD28 superagonists: mode of action and therapeutic potential. Immunol Lett 100:21-28.
13. Hünig, T. 2006. Human CD28 specific monclonal antibodies for antigen-non-specific activation of T-lymphocytes. T. GmbH, editor. USA.
14. Siefken, R., Kurrle, R., and Schwinzer, R. 1997. CD28-mediated activation of resting human T cells without costimulation of the CD3/TCR complex. Cell Immunol 176:59-65.
15. Singh, M., Basu, S., Camell, C., Couturier, J., Nudelman, R.J., Medina, M.A., Rodgers, J.R., and Lewis, D.E. 2008. Selective expansion of memory CD4(+) T cells by mitogenic human CD28 generates inflammatory cytokines and regulatory T cells. Eur J Immunol 38:1522-1532.
16. Romer, P.S., Berr, S., Avota, E., Na, S.Y., Battaglia, M., ten Berge, I., Einsele, H., and Hunig, T. 2011. Preculture of PBMCs at high cell density increases sensitivity of T-cell responses, revealing cytokine release by CD28 superagonist TGN1412. Blood 118:6772-6782.
17. Suntharalingam, G., Perry, M.R., Ward, S., Brett, S.J., Castello-Cortes, A., Brunner, M.D., and Panoskaltsis, N. 2006. Cytokine storm in a phase 1 trial of the anti-CD28 monoclonal antibody TGN1412. N Engl J Med 355:1018-1028.
18. Hunig, T. 2007. Manipulation of regulatory T-cell number and function with CD28-specific monoclonal antibodies. Adv Immunol 95:111-148.
19. Brinkmann, V., Kinzel, B., and Kristofic, C. 1996. TCR-independent activation of human CD4+ 45RO- T cells by anti-CD28 plus IL-2: Induction of clonal expansion and priming for a Th2 phenotype. J Immunol 156:4100-4106.
20. Luhder, F., Huang, Y., Dennehy, K.M., Guntermann, C., Muller, I., Winkler, E., Kerkau, T., Ikemizu, S., Davis, S.J., Hanke, T., et al. 2003. Topological requirements and signaling properties of T cell-activating, anti-CD28 antibody superagonists. J Exp Med 197:955-966.
21. Miyara, M., Yoshioka, Y., Kitoh, A., Shima, T., Wing, K., Niwa, A., Parizot, C., Taflin, C., Heike, T., Valeyre, D., et al. 2009. Functional delineation and differentiation dynamics of human CD4+ T cells expressing the FoxP3 transcription factor. Immunity 30:899-911.
22. Beyersdorf, N., Hanke, T., Kerkau T., and Hunig, T., 2005. Superagonistic anti-CD28 antibodies: potent activators of regulatory T cells for the therapy of autoimmune diseases. Ann Rheum Dis. 2005 November; 64(Suppl 4): iv91-iv95.

## Claims

1. An *in vitro* method for the expansion of T cells comprising the steps of
a) providing conventional anti-CD28 monoclonal antibodies immobilized on solid beads;
b) providing T cells from a donor;
c) reacting the T cells with the immobilized conventional anti-CD28 monoclonal antibodies under suitable conditions thereby expanding the T cells.

2. The method of claim 1, wherein the solid beads are selected from magnetic beads, preferably superparamagnetic polystyrene beads.

3. The method of claim 1 or 2, wherein the beads have a diameter of about 0.5-10 µm, preferably 4-5 µm.

4. The method according to one or more of the preceding claims, wherein the conventional anti-CD28 monoclonal antibody is indirectly immobilized to said beads, preferably via another antibody coated on said beads.

5. The method according to one or more of the preceding claims, where the conventional anti-CD28 monoclonal antibody is binding to an epitope on CD28 molecules outside their C"D loop.

6. The method according to one or more of the preceding claims, wherein the expanded T cells are CD4⁺ T cells.

7. The method according to one or more of the preceding claims, wherein the T cells of step b) are derived from peripheral blood of a human donor.

8. The method according to one or more of the preceding claims, wherein said T cells expanded in step c) are further processed to a form suitable for parenteral, preferably intravenous, administration to said donor.

9. The method according to one or more of the preceding claims, wherein the beads are coated with a monoclonal or polyclonal anti-mouse Ig antibody.

10. A solid bead having a conventional anti-CD28 monoclonal antibody immobilized thereto, wherein the bead is free of anti-CD3 antibodies.

11. The bead of claim 10, where the bead is coated with an anti-mouse Ig monoclonal antibody to which the conventional anti-CD28 monoclonal antibody is bound.

12. A coated bead, consisting of a solid bead, an anti-mouse Ig monoclonal antibody and a conventional anti-CD28 monoclonal antibody.

13. A kit consisting essentially of a bead of one or more of claims 10-12.

14. The use of a conventional anti-CD28 monoclonal antibody in the *in vitro* expansion of T cells, where the conventional anti-CD28 monoclonal antibody is the only anti-CD antibody used.
